# EUROPEAN PATENT APPLICATION

(11) **EP 1 681 568 A1**
(43) Date of publication of application: **19.07.2006**
(21) Application number: 04792372.7
(22) Date of filing: 14.10.2004
(51) Int. Cl.: G01N 33/531, C07K 7/06, C07K 7/08, C07K 14/00

(54) **ARTIFICIAL ANTIBODY COMPRISING COMPLEMENTARY PEPTIDE**

(30) Priority: 29.10.2003 JP 2003368875
(71) Applicant: Okada, Hidechika, Nagoya-shi, Aichi 467-0803 (JP); Okada, Noriko, Nagoya-shi, Aichi 467-0803 (JP)
(72) Inventor: Okada, Hidechika, Nagoya-shi, Aichi 467-0803 (JP); Okada, Noriko, Nagoya-shi, Aichi 467-0803 (JP)
(74) Representative: Becker Kurig Straus
(86) International application number: PCT/JP2004/015140
(87) International publication number: WO 2005/040799

(57) **Abstract**

It is intended to design and synthesize a peptide complementary to the target site of an antigen protein, confirm the ability thereof to bind to the target site and thus provide an artificial peptide for detecting the antigen protein. Namely, an artificial antibody peptide wherein the specific binding of a complementary peptide, which has been constructed by using, for example, a designing program MIMETIC for automatically designing a complementary peptide corresponding to the amino acid sequence of a peptide at the target site, to the target site is confirmed to give an artificial antibody peptide.

## Description

### BACKGROUND OF THE INVENTION

### Field of the Invention

The invention relates to a method for detecting a target protein using a complementary peptide, which is an artificial antibody as a substitute of a natural antibody, prepared by using an art for designing the complementary peptide having binding affinity to proteins, and a method for analyzing the target protein.

### Description of the Related Art

One of evaluation criteria for utilizing a complementary peptide by a genetic algorithm (genetic evolution method) is a hydrophobicity value. A mean value of the hydrophobicity value is calculated for amino acids at each site of the peptide by adding amino acids in front of and at the back of the site (five or several amino acids in front of and at the back of the site; zero amino acid for a terminal amino acid), and the hydrophobicity value is evaluated by a value having an inverse sign of the mean value. For example, when the mean value is +3.0, a value of -3.0 is evaluated as a full mark of the hydrophobicity value at that amino acid site. A second evaluation index is correspondence of bulkiness of an amino acid side chain at a corresponding site. It is evaluated that an a-amino carbon (a carbon atom of a group to which the side chain of the amino acid is linked) of the a corresponding amino acid has side chain bulkiness that does not interfere with another amino acid to come to a distance of 5 ? . Correspondence is evaluated as a full mark when the side chain does not interfere with another side chain to approach due to its bulkiness, and the complementary peptide is evaluated by subtraction depending on the extent of interference. A third evaluation index is coincidence of backbone alignment of a peptide frame, and the complementary peptide is evaluated by coincidence of the backbone alignment. A give number of peptides (for example 300 peptides) designed so as to have the same number of amino acids as the target peptide are generated as candidate peptide libraries, and complementarity against the target peptide is evaluated to store the evaluated value in a computer memory. Two higher-ranking peptides having higher evaluation scores are selected in the peptide library when a given number of evaluation peptide libraries are obtained, and the peptide libraries of the next generation are produced by modifying the amino acid sequences of these peptides by scrambling, or by designing other candidate peptides by arbitrarily substituting the amino acids. Complementarity against the target peptide is evaluated for each peptide to store the evaluated value in the computer memory, and two higher-ranking peptides are selected to produce next-to-next generation peptide libraries by the same method as described above. These procedures are repeated, and are ideally continued to repeat until full peptides of full mark are obtained. A list is created by aligning the record of evaluation of respective peptides by sorting in the order of higher ranking, and peptides having higher ranking (for example higher 300 peptides) are stored as a list in the record. This computer program software is called as MIMETIC. Peptides having full mark or almost full mark are synthesized, and binding affinity to proteins having the target peptide is evaluated in order to employ peptides having higher binding affinity as artificial antibody peptides.

Alternatively, an antigen is immunized to mouse or rabbit, and an antibody may be purified from a serum using the serum of an antigen-producing animal as an anti-serum. Hybridoma is produced by fusing lymphocytes, which are obtained from spleen cells of an animal such as mouse immunized with the antigen, with a myeloma cell strain, and the desired antibody-producing hybridoma is cloned to allow it to produce a monoclonal antibody. However, it is only good luck that the animal recognizes the desired epitope and produces the antibody. While a phage display method is used for producing a reactive antigen against an epitope having no antigenicity against the immunized animal, this is a quite complicated method.

### SUMMARY OF THE INVENTION

Accordingly, it is an object of the invention to design a complementary peptide that is expected to be reactive to a target peptide portion of an antigen protein using a computer program such as MIMETIC. Another object of the invention is to enable the complementary peptide to be used as an artificial antibody peptide by synthesizing the designed peptide in order to confirm specific binding affinity of the peptide.

The invention provides a method for designing a peptide being complementary to an amino acid sequence of a target peptide portion of an antigen protein using a computer program. The invention also provides an artificial antibody peptide having a high specific binding affinity by allowing the synthesized peptide to react with the target protein in order to investigate specific binding affinity to the target protein.

The complementary peptide that binds to an amino acid sequence of a target site of an arbitrary protein is produced in the invention, and the peptide is used as an artificial antibody peptide for detecting the antigen protein. A design program software MIMETIC can be used for designing the complementary peptide. Since the artificial antibody peptide reactive to an arbitrary site of the protein selected as a target can be freely designed, no complicated methods for producing antibodies by immunizing animals are required. In addition, since an artificial antibody peptide against a novel protein can be promptly produced without preparing any antibodies in advance, the method of the invention is able to readily construct a method for detecting novel proteins. Antibodies against common sites of proteins among animal species can be hardly expected to be produced by immunizing animals for antibody production. However, since the method for producing the artificial antibody peptide of the invention is able to create a peptide reactive to the common site among the animal species, a method for detecting antigen proteins may be readily constructed. When a detection system such as an ELISA method and a protein array method that utilize two or more antibodies is to be constructed, the method of the invention is quite useful because any artificial antibody peptides against separated cites of a target molecule can be designed and produced.

### DESCRIPTION OF THE PREFERRED EMBODIMENTS

### (Example 1)

Complementary peptides against peptides at each site of reverse transcriptase (abbreviated as RT hereinafter) of a pathogenic virus of AIDS, human immunodeficiency virus-1 (HIV-1), were automatically designed using an analysis program (MIMETIC), and the peptides shown in Table 1 were designed. The designed peptides were synthesized by a conventional solid phase method by allowing 9-fluorenylmethoxycarbonyl (Fmoc) amino acids to sequentially bind using a peptide synthesizer (trade name: AMS 422 Multiple Peptide Synthesizer, manufactured by ABiMED Co.). The synthesized peptide was released from the solid support by a conventional method, and protective groups were removed. The peptide obtained was recovered by ether precipitation, and was purified with reversed phase HPLC after removing ether by drying. A culture supernatant of PLB cells (established human lymphocyte strain) infected with HIV-1 was placed on a top layer of layered 65% and 15% sucrose solutions in a ultracentrifuge tube, and HIV-1 particles were retrieved by ultracentrifugation for 1 hour at 26,500 rpm. Virus RNA was extracted by a guanidine isothiocyanate method (Chomezynski, P. and Sacchi, N., RNA Isolation from Cultured cells, Analytical Biochemistry, 162: 156-159, 1987), and the extract was used by dissolving in 5 mM Tris buffer (pH 7.5). The amount of the whole length virus genome RNA contained in the total RNA sample was quantified according to a conventional ribonuclease protection method (Kaye, J. F. et al., Cis-acting Sequences Involved in Human Immunodeficiency Virus Type 1 RNA Packaging, J. Virol, 69: 6588-6592, 1995; and Huang, Y. et al., The Role of Nucleocapsid and U5 stem/A rich Loop Sequences in tRNA (3Lys) Genomic Placement and Initiation of Reverse Transcription in Human Immunodeficiency Virus Type 1, J. Virol., 72: 3907-3915, 1997). The virus genome binds to tRNA^{Lys3} in the cell, and can be used for measuring RT activity. The RNA genome of about 5 × 10⁷ molecules was allowed to react with 50 ng of HIV-RT, 10 units of RNase and dNTP's (deoxynucleic acid) at 37° C for 15 minutes in 20 µl of RT buffer (50 mM Tris-HC1, pH 7.5, 60 mM KC1, 3 mM MgCl₂ and 10 mM DTT). The sequence at the tip of the molecule comprises six bases of CTGCTA. One base was added to tRNA^{Lys3} using ³²P-dCTP with a radioactivity of 5 µCi, and 6 bases were further added by allowing 0.2 mM of dCTP, 0.2 mM of dTTP, 5 µCi of ³²P₋dGTP and 0.05 mM of ddATP to react. The elongated primer was retrieved by ethanol precipitation, and was analyzed by auto-radiography through electrophoresis on 6% polyacrylamide gel containing 7 M urea. A test peptide for investigating an action against RT activity was added to the RT reaction system, and the presence, if any, and amount of the added base sequences were assayed. The results showed that three peptides of TLMA2993, PSTW1594 and ESLA2340 suppress 50% of the RT activity at a concentration of about 20 pM. While no suppressing activity was observed in the remaining 7 peptides, 30% of the designed peptides showed the suppressing activity. This result indicates that peptides having the desired activity could be designed in a quite high probability. The results are summarized in Table 1. Table 1 shows amino acid sequences of various sites considered to be involved in the activity of reverse transcriptase (RT), and amino acid sequences of the peptides automatically designed for the sites using MIMETIC. Of 10 peptides, 3 peptides suppressed the RT activity.

**TABLE 1**

| Target Peptide in RT Molecule | Sub-Domain in RT Molecule | Automatically Designed Complementary Peptide (Name of Peptide) |
|---|---|---|
| 96HPAGLKKKKSVTVLDVGDAY115 | palm | MWATELILISDSDEVDSIQM (MWAT2299) |
| 178PDIVIYQYMDDLYVGSDLEI191 | palm | PFYPMIHIHHVGVKSDIEVY (RFYP2488) |
| 283LRGTKALTEVIPLTEEAELEL302 | thumb | ESLALYKSLQQSEMLLELEL (ESLA2340) ESLALYKSLQ (ESLA1153) QSEMLLELEL (QSEM1205) |
| 383WGKTPKFKLPIQKETWETWWTEYW QATWIPE413 | connection | TLMALELKGKLLLAGLAPSAFLPLSFPEL (TLMA 2993) |
| | | PSTTPTFLKFQLK (PSTT1508) |
| | | PSTWPTFLKFQLK (PSTW1594) |
| | | IPARLGHMFMLRRVGL (IPAR1900) |
| 350KTGKYARMRGAHTN363 | connection | LSATMAAAAASMS (LSAT 1256) |

| | | |
|---|---|---|
| 50% suppression concentrations against RT were 17 pM for ESLA2340, 15 pM for TLMA 2993 and 15 pM for PSTW 1594. No suppression action against RT was observed in other complementary peptides. | | |

HIV transcriptase (HIV-RT) was detected as follows using ESLA 2340 and TLMA 2933 that were confirmed to suppress the activity of transcriptase, and using these complementary peptides as artificial antibody peptides. Wells of a 96 well plate were coated with ESLA 2340 in advance. After adding a diluted solution of HIV-RT in each well and allowing the plate to stand overnight at 4° C, the plate was washed and biotin-labeled TLMA 2993 was added to each well. The plate was allowed to stand for 1 hour at room temperature. After washing the plate again, peroxidase-labeled avidin was added to allow it to react at room temperature for 1 hour. After washing the plate to remove unreacted avidin, a color development reaction was performed by a conventional method at room temperature by adding a color developing reagent of peroxidase. A color was developed depending on the concentration of HIV-RT, which shows that the artificial peptide antibody can be used for a sandwich ELISA method.

### (Example 2)

Pro-carboxypeptidase R (abbreviated as ProCPR hereinafter) is converted into active carboxypeptidase R (abbreviated as CPR hereinafter) by trimming a sequence from an amino terminal to arginine 92 with a trypsin-like enzyme such as thrombin and plasmin. A complementary peptide corresponding to the amino acid sequence comprising 30, 24, 20, 15 or 11 amino acids from amino acid 87 counted from the amino terminal of the ProCPR was automatically designed using the analysis program MIMETIC of the invention, and the peptides shown in Table 2 were designed. An action on the activation reaction of ProCPR by a thrombin-thrombomodulin complex (abbreviated as T/TM complex hereinafter) was analyzed with respect to the peptides (the peptides in notes (1) and (2) in Table 2) comprising 20 and 15 amino acids of the complementary peptides described above. The designed peptides were synthesized by a conventional solid state method by which 9-fluorenylmethoxycarbonyl (Fmoc) amino acids are sequentially linked using a peptide synthesizer (trade name: AMS 422 Multiple Peptide Synthesizer, manufactured by ABiMED Co.). The synthesized peptide was released by a conventional method, and protecting groups were removed. The peptide obtained was retrieved by ether precipitation, and was purified by reversed phase HPLC after removing ether by drying. The purified peptides comprising 20 and 15 amino acids were allowed to react with ProCPR for 10 minutes at room temperature, followed by allowing the T/TM complex to react. After a reaction for 45 minutes at 37°C by adding hippuril-L-arginine which is substrate of CPR, free hippuric acid was measured according to a reported method (Komura, H., et al. , Effect of Anticoagulants in Colorimetric Assay for Basic Carboxypeptidase, Microbiol. Immunol., 46: 115-117, 2002). When 20-mer peptide and 15-mer peptide were added, activation of ProCPR to CPR with the T/TM complex was suppressed by adding 1 µM of respective peptides. The fact that both two peptides investigated exhibited a suppression effect on ProCPR as a target seems to indicate good efficiency of the complementary peptide design program software that is named as MIMETIC by us. While ProCPR is activated with elastase and trypsin, these peptides suppressed only activation by the T/TM complex, and did not inhibit activation by elastase and trypsin. Complementary peptide corresponding to designed ProCPR is shown in table 2. Table 2 shows mimetic peptides that were automatically designed by MIMETIC corresponding to the amino aced sequence comprising 11, 15, 20, 24 and 30 amino acids at the downstream of amino acid 87 of ProCPR. Since a sugar chain is conjectured to be added to asparagine 86, this amino acid was omitted and the peptide comprising amino acid 87 and thereafter was targeted. Since the carboxyl side of arginine 92 is located at the cleavage site with thrombin, a mimetic peptide was designed so as to step over arginine 92. Two peptides comprising 15 and 20 amino acids were selected in order to investigate the action of the peptide on activation of ProCPR with the T/TM complex, and found that both peptides showed an inhibitory effect at a concentration of 1 µM.

**TABLE 2**

| Position in ProCPR Molecule | Amino Acid Sequence | Complementary Peptide (Mimetic Peptide) |
|---|---|---|
| 87-97 (11aa) | DTVSPRASASY | VSGRKRRAGIR |
| 87-101 (15aa) | DTVSPRASASYYEQY | VSRGRRRDRRILMII (NOTE 1) |
| | | VRSGRTRARRLILII |
| | | VSGRRRRDRIRLMII |
| 87-106 (20aa) | DTVSPRASASYYEQYHSLNE | VGGRRTRARRVLLLVLTETH (NOTE 2) ISGSRRRATTWDKRVKAEGL |
| 87-110 (24aa) | DTVSPRASASYYEQYHSLNEIYSW | ISGGTLTARACFLLIHTEVLDVTP |
| 87-116 (30aa) | DTVSPRASASYYEQYHSLNEIYSWIE FITE | VCGEGISARAWELWGRILNSAPYF QYPL |

| | | |
|---|---|---|
| (Note 1) and (Note 2) are peptides that are confirmed to suppress activation of ProCPR by actually synthesizing the peptides. | | |

VGGRRTRARRVLLLVLTETH (abbreviated as VGG hereinafter) comprising 20 amino acids, which was confirmed to suppress activation of ProCPR, was confirmed to be reactive to ProCPR using a surface plasmon resonance analyzer (manufactured by Biacore Co.). After coating the plate with VGG and washing the plate, diluted human plasma containing ProCPR was added to the plate and was allowed to react for 1 hour at room temperature. After removing excess plasma by washing the plate, 10G1 as a monoclonal antibody against biotin-labeled ProCPR was added followed by allowing it to react for 1 hour at room temperature. Excess 10G1 antibody was removed by washing the plate, and remaining 10G1 antibody was allowed to react by adding peroxidase-labeled avidin. After removing unreacted avidin by washing, the reaction solution was made to develop a color by a conventional method depending on the strength of the enzyme reaction of peroxidase by adding a peroxidase color development reagent, and the intensity of the color was measured with a plate reader. Since the color was developed depending on the concentration of the plasma containing ProPCR, it could be confirmed that VGG coated on the plate can be used as an artificial peptide antibody.

## Claims

1. A method for detecting an antigen using a complementary peptide reactive to a target protein as an artificial antibody.

2. A complementary peptide reactive to the target protein according to Claim 1 labeled with an enzyme, a radioisotope, biotin or avidin.

3. A kit containing the complementary peptides using at least two complementary peptides against the same antigen according to Claims 1 and 2, one of the complementary peptides being immobilized on a solid phase, and an antigen reactive to said complementary peptide or another complementary peptide reactive to said antigen being labeled with an enzyme, a radioisotope or biotin.

4. The kit according to Claim 3 comprising a complementary peptide and a natural antibody recognizing an antigen.
